Europäisches Patentamt

European Patent Office

Office européen des brevets

(19)

(11) **EP 0 894 268 B1**

(12) **FASCICULE DE BREVET EUROPEEN**

(45) Date de publication et mention
de la délivrance du brevet:
**27.07.2005 Bulletin 2005/30**

(21) Numéro de dépôt: **97918188.0**

(22) Date de dépôt: **09.04.1997**

(51) Int Cl.[7]: **G01N 33/28**, B01F 15/04

(86) Numéro de dépôt international:
**PCT/FR1997/000627**

(87) Numéro de publication internationale:
**WO 1997/039349 (23.10.1997 Gazette 1997/45)**

(54) **PROCEDE ET DISPOSITIF DE PREPARATION D'UN CARBURANT, NOTAMMENT POUR MOTEUR DIESEL, PAR MELANGE EN LIGNE DE SES CONSTITUANTS**

VERFAHREN UND VORRICHTUNG ZUM AUFBEREITEN EINES BRENNSTOFFES, INSBESONDERE FÜR DIESELMOTOREN, DURCH IN-LINE MISCHUNG SEINER BESTANDTEILE

METHOD AND DEVICE FOR PREPARING A FUEL, PARTICULARLY FOR DIESEL ENGINES, BY ON-LINE MIXTURE OF ITS COMPONENTS

(84) Etats contractants désignés:
**AT BE DE ES FI FR GB IT NL PT SE**

(30) Priorité: **15.04.1996  FR 9604656**

(43) Date de publication de la demande:
**03.02.1999  Bulletin 1999/05**

(73) Titulaire: **TotalFinaElf France
92800 Puteaux (FR)**

(72) Inventeurs:
  • **RENAULT, François
    F-76620 Le Havre (FR)**
  • **LEBRETON, Daniel
    F-78230 Le Pecq (FR)**
  • **DROUAULT, Jean-Pierre
    F-76700 Harfleur (FR)**
  • **PICART, Alain
    F-76700 Harfleur (FR)**

(74) Mandataire: **Jolly, Jean-Pierre et al
Cabinet Jolly
54, rue de Clichy
75009 Paris (FR)**

(56) Documents cités:
**EP-A- 0 610 118          DE-B- 1 270 838
US-A- 3 469 954          US-A- 3 520 173
US-A- 4 010 358**

• **MEASUREMENT AND CONTROL, vol. 3, no. 1, Janvier 1970, LONDON GB, pages 19-23, XP002020139 E.B. JONES, ET AL: "ON-LINE OCTANE MEASUREMENT AND ITS INFLUENCE ON BLENDING PLANT DESIGN AND OPERATION"**
• **DATABASE WPI Week 8829 Derwent Publications Ltd., London, GB; AN 88-202111 [29] XP002020140 & JP 63 139 988 A (NIPPON MINING KK) , 11 Juin 1988**

## Description

La présente invention concerne un procédé et un dispositif pour la préparation d'un carburant, notamment pour moteur diesel, ou d'un combustible pour installation de chauffage, par mélange en ligne de ses constituants, avec éventuellement incorporation d'additifs.

Les carburants pour moteur diesel sont destinés à s'auto-inflammer après injection dans de l'air fortement comprimé. Ils sont généralement constitués d'un mélange de divers composants, dans des proportions telles que le carburant présente certaines qualités et réponde à certaines spécifications relatives, par exemple, à la tenue au froid, la teneur en soufre et la valeur du cétane.

Dans les raffineries, un tel carburant est réalisé par la technique dite du mélange en ligne, selon laquelle les différents constituants liquides ainsi que les additifs sont introduits simultanément et en continu dans une même conduite servant de mélangeuse. Le débit des différents constituants est commandé et contrôlé par un ordinateur et la durée de préparation d'un lot de carburant peut s'étendre sur 30 heures.

Naturellement, les propriétés du produit résultant sont contrôlées à diverses reprises en cours de fabrication et des analyses sont effectuées dans ce but sur des échantillons prélevés en sortie de la mélangeuse, ou dans le bac de stockage en cours de remplissage. A partir des résultats de ces analyses, les débits des constituants du mélange ainsi que des additifs sont ajustés pour aligner les valeurs mesurées avec les valeurs de consigne.

Bien entendu, pour chaque analyse, il y a avantage à limiter autant que possible l'intervalle de temps séparant le prélèvement d'un échantillon du mélange en cours de préparation et l'obtention de la valeur mesurée, et les analyseurs utilisés dans ce but, dits analyseurs en ligne, répondent en général à cette exigence.

L'une des propriétés essentielles des carburants pour moteur diesel, à savoir leur aptitude à l'auto-inflammation, ne fait cependant pas l'objet d'un contrôle en ligne à la sortie de la mélangeuse, en raison des difficultés de mesure de cette caractéristique. Celle-ci est définie comme la durée séparant l'injection du carburant et son inflammation dans une chambre de combustion, dans les conditions fixées par la norme ASTM D 613, où l'unité de mesure est conventionnellement dénommée nombre de cétane.

La valeur 15 de ce nombre de cétane est attribuée, dans les conditions de la mesure, à l'heptaméthylnonane, tandis que la valeur 100 correspond à la combustion dans les mêmes conditions, du cétane normal (hexadécane normal). Un nombre intermédiaire CN (de l'anglais Cetane Number) attribué à un carburant signifie que celui-ci présente une auto-inflammation équivalente à celle d'un mélange d'heptaméthylnonane et de cétane normal tel que

$$CN = X + 0,15(100 - X)$$

où X désigne la teneur en cétane (% en volume) du mélange heptaméthylnonane et cétane normal.

La mesure, longue et compliquée, s'effectue dans des conditions bien précises, fixées par la norme ASTM D 613, avec un appareillage défini dans la même norme. Cet appareillage comporte un moteur diesel mis au point par la société Corporation Fuel Research et universellement connu dans la technique sous le nom de " moteur C.F.R. ". La procédure de mesure avec cet appareillage est toutefois longue (de l'ordre d'une heure), délicate et nécessitant de nombreuses interventions manuelles

Des procédés et des dispositifs ont été mis au point pour diminuer la durée de la mesure tout en rendant automatique cette dite mesure. En particulier, la demande de brevet Européen EP-A- 0 610 118, qui reprend la procédure telle que décrite dans le document SAE 890419, propose un procédé et un dispositif pour la mesure de l'indice de cétane en continu. Si ce procédé et ce dispositif apportent une amélioration à la méthode de mesure du cétane d'un carburant, ils présentent toutefois des inconvénients, tels que l'utilisation de circuits et capacités communes pour le produit de référence et l'échantillon du carburant en cours de fabrication, ainsi que la nécessité d'utiliser de nombreuses électrovannes avec leurs systèmes de pilotage.

D'autres méthodes existent pour mesurer le délai d'auto-inflammation d'un carburant, telles que le calcul de l'indice de cétane à partir de propriétés aisément mesurables, comme la densité et les caractéristiques de distillation. Les résultats obtenus sont toutefois peu fiables et ils ne tiennent pas compte de l'introduction fréquente dans les carburants d'un additif procétane, destiné à augmenter, si nécessaire, le nombre de cétane du mélange. Il est intéressant de noter, à ce propos, que le moteur C.F.R., lui, prend en compte l'effet de l'additif procétane dans ses mesures.

Un besoin se fait donc sentir, dans la technique, d'un moyen de mesure rapide, fiable et automatique, utilisé en ligne dans le processus de préparation d'un carburant pour moteur diesel, permettant de mieux contrôler la caractéristique d'auto-inflammation représentée par le nombre de cétane.

C'est ce problème que se propose de résoudre la présente invention, par utilisation en ligne, en aval de la mélangeuse, d'un appareillage du type C.F.R., dans des conditions telles que les résultats de la mesure du nombre de cétane puissent être connus facilement et à bref délai après le prélèvement de l'échantillon à tester.

L'invention vise également à proposer un procédé et un dispositif de mélange en ligne des constituants, et éventuellement des additifs, d'un carburant pour moteur diesel, dans lesquels la mesure du nombre de cétane du mélange réalisé puisse être rapide, fiable et entièrement automatisée.

A cet effet, la présente invention a pour objet un procédé de préparation d'un carburant, notamment pour moteur diesel, ou d'un combustible pour installation de chauffage, par mélange en ligne de ses constituants avec éventuellement incorporation d'additifs, ce carburant devant posséder en vue de sa commercialisation un nombre de cétane spécifique, procédé dans lequel on alimente une mélangeuse en continu avec les différents constituants à des débits commandés, on mesure, à la sortie de la mélangeuse et à différents intervalles de temps, le nombre de cétane du carburant en cours de préparation, on calcule l'écart de ce nombre de cétane par rapport à au moins une valeur de consigne, puis on ajuste les débits respectifs des différents constituants ou d'additifs de manière à annuler l'écart entre les valeurs mesurées et les valeurs de consigne, ce procédé étant caractérisé en ce que l'on alimente un moteur de type " C.F.R. " alternativement avec un carburant en cours de fabrication, prélevé sur la ligne d'évacuation de la mélangeuse, puis avec un produit de référence dont le nombre de cétane est connu, par l'intermédiaire de deux circuits d'alimentation distincts possédant chacun une pompe d'injection haute pression, le dit moteur C.F.R. fonctionnant avec un taux de compression constant.

Le calcul du nombre de cétane de l'échantillon à mesurer, par comparaison avec le nombre de cétane déjà connu d'un produit de référence, s'effectuera de façon simple en appliquant les dispositions de la norme ASTM D 2885 pour la partie analyseur en ligne, déjà utilisée pour déterminer l'indice d'octane des essences. Dans la pratique, on mesure séquentiellement, dans les mêmes conditions et à l'aide d'un même moteur C.F.R., le délai d'inflammation d'un échantillon du mélange en cours de fabrication, puis le délai d'inflammation d'un produit de référence ayant un nombre de cétane connu. La durée de ces mesures sur le carburant en cours de fabrication, puis sur le produit de référence, est inférieure à une heure, voire inférieure à dix minutes.

Le nombre de cétane de l'échantillon du mélange en cours de fabrication sera déterminé, pour chaque série de mesures, à partir des informations fournies sous forme de signaux électriques par le moteur C.F.R. (délais d'auto-inflammation de l'échantillon-du mélange et du produit de référence), ces informations étant ensuite converties, par l'intermédiaire d'un calculateur, en écarts de cétane par rapport au produit de référence, ledit calculateur calculant ensuite, en fonction du nombre de cétane connu du produit de référence, le nombre de cétane de l'échantillon à mesurer.

Ce nombre de cétane de l'échantillon sera ensuite transmis à un ordinateur programmé pour piloter les quantités relatives des différents constituants introduits dans la mélangeuse.

La précision de la mesure du nombre de cétane par le dispositif conforme à l'invention est, conformément à la norme ASTM D 2885, directement liée à la précision du nombre de cétane du produit de référence, cette précision étant généralement égale ou inférieure à 1 point de cétane pour un produit de référence ayant fait l'objet de plusieurs mesures dont la valeur moyenne est égale à 50. Avantageusement, la précision de la mesure du nombre de cétane par le dispositif conforme à l'invention est donc améliorée d'un facteur pouvant varier entre deux et cinq, par rapport à la précision de la méthode décrite dans la norme ASTM D 613.

on notera que le moteur C.F.R. est utilisé, dans le procédé de l'invention, à taux de compression constant et, par conséquent, dans des conditions différentes de celles de la norme ASTM D 613, qui préconise un taux de compression variable et implique une comparaison avec deux produits de référence parfaitement définis, ayant des nombres de cétane respectivement supérieur et inférieur à celui de l'échantillon. L'utilisation du moteur C.F.R. à taux de compression constant évite d'avoir recours à des mouvements d'organes mécaniques au cours des mesures et ceci est donc favorable à une automatisation du procédé de mesure, donc à une amélioration de la précision des mesures.

Le produit de référence utilisé aura un nombre de cétane proche de celui recherché pour le mélange en cours de fabrication, et dont l'écart avec la valeur de consigne est inférieur à 5 points de cétane et de préférence inférieur à 2.

L'échantillon du mélange et le produit de référence qui alimentent le moteur C.F.R. sont introduits par l'intermédiaire de circuits distincts dans la même zone d'injection du dit moteur, ce qui évitera d'avoir à effectuer une purge entre les deux mesures successives, permettant ainsi de diminuer le temps de stabilisation des dites mesures. Ces deux mesures (échantillon du mélange et produit de référence) pourront être effectuées dans un ordre quelconque.

Chaque circuit d'alimentation du moteur C.F. R. sera équipé d'une pompe d'injection haute pression distincte, ce qui constitue un avantage important du procédé conforme à l'invention, en évitant l'utilisation de systèmes commandés pour, par exemple, déclencher l'ouverture et la fermeture d'électrovannes, réaliser des réglages intermédiaires entre les mesures sur une unique pompe, ou déclencher le rinçage de la dite pompe lors du passage du produit de référence au carburant en cours de fabrication. Un unique pré-réglage étant effectué pour les deux circuits avant la mise en oeuvre du procédé, pour un cycle de fabrication.

Le système de mesure sera donc facile à automatiser, puisqu' il suffira de commander alternativement une pompe de l'un ou l'autre des circuits d'alimentation du moteur C.F.R.

Une addition en ligne, en quantités réglables, d'un additif de procétane dans la mélangeuse, ou en amont ou en aval de celle-ci, peut être effectuée, comme dans la technique antérieure, mais, ainsi qu'il a été indiqué ci-dessus, les mesures effectuées par le dispositif C.F.R. tiennent compte de ce procétane, ce qui constitue un avantage important du procédé conforme à l'invention.

L'invention a également pour objet un dispositif pour la fabrication d'un carburant, notamment pour moteur diesel, ou un combustible pour installation de chauffage, par mélange en ligne de ses constituants avec éventuellement incorporation d'additifs, ce carburant devant posséder en vue de sa commercialisation un nombre de cétane spécifique, ce dispositif comprenant au moins deux bacs pour le stockage d'au moins deux constituants du mélange, une mélangeuse alimentée par des lignes distinctes à débits réglables en les divers constituants du mélange, au moins une ligne d'évacuation du mélange réalisé dans la mélangeuse, de préférence un bac pour le stockage du produit fabriqué, éventuellement une source d'additifs de procétane à débit réglable et automatisé, connectée en amont ou en aval à la mélangeuse et/ou à la ligne d'évacuation, un moyen de mesure du nombre de cétane, au moins un moyen de commande du débit des diverses lignes d'alimentation de la mélangeuse et de la ligne d'alimentation en procétane, ce moyen de commande étant d'une part, asservi au moyen de comparaison et d'autre part, programmé de manière à ajuster les différents débits pour réduire l'écart entre les valeurs mesurées et les valeurs du nombre de cétane de consigne, ce dispositif étant caractérisé en ce que :

- le moyen de mesure destiné à mesurer le nombre de cétane du mélange comprend un moteur du type C.F.R., monté de préférence en dérivation sur la ligne d'évacuation du mélange,
- ce moteur C.F.R. est connecté respectivement par des lignes, équipées d'un moyen d'obturation commandé, à ladite ligne d'évacuation et à une source d'un produit de référence ayant un nombre de cétane connu,
- le moteur C.F.R. est pré-réglé pour fonctionner à taux de compression constant et avec une avance à l'injection réglée sur une valeur prédéterminée,
- le moteur C.F.R. est apte à fournir au moyen de commande des débits des différentes lignes d'alimentation en constituants du mélange, ou éventuellement en additif, un signal représentatif de la différence entre deux mesures du nombre de cétane, les dites mesures étant effectuées successivement sur le mélange et sur le produit de référence ou inversement.

Comme indiqué ci-dessus, les lignes d'alimentation du moteur C.F.R. en échantillon du mélange à tester et en produit à nombre de cétane connu seront, de préférence, distinctes ou ne comprendront qu'une faible partie commune, et elles seront équipées chacune d'une pompe d'injection. Des clapets anti-retour pourront naturellement être prévus sur les lignes d'alimentation du moteur C.F.R.

Avantageusement, les pompes d'injection seront pilotées par une commande automatique apte à les actionner à intervalles réguliers préfixés, par exemple toutes les cinq minutes.

De façon connue en soi, les pompes d'injection, dans le moteur C.F.R., de l'échantillon du mélange et du produit de référence, comprendront deux systèmes de réglage, l'une de la quantité à injecter, l'autre de l'instant de l'injection, et ces deux systèmes pourront faire l'objet d'une régulation indépendante.

Le moteur C.F.R. sera avantageusement étalonné à l'aide de deux carburants étalons ayant des nombres de cétane encadrant celui du produit de référence.

Les dessins schématiques annexés illustrent la mise en oeuvre de l'invention. Sur ces dessins n'ont été représentés que le système de mesure et de régulation du nombre de cétane, et non les systèmes d'un type connu utilisés soit pour étalonner un appareil de mesure, soit pour mesurer et réguler d'autres propriétés préfixées du carburant. Sur ces dessins :

La figure 1 est un schéma de l'ensemble du dispositif de mélange en ligne des constituants du carburant ;
La figure 2 illustre le système de mesure du nombre de cétane du mélange en cours de réalisation.

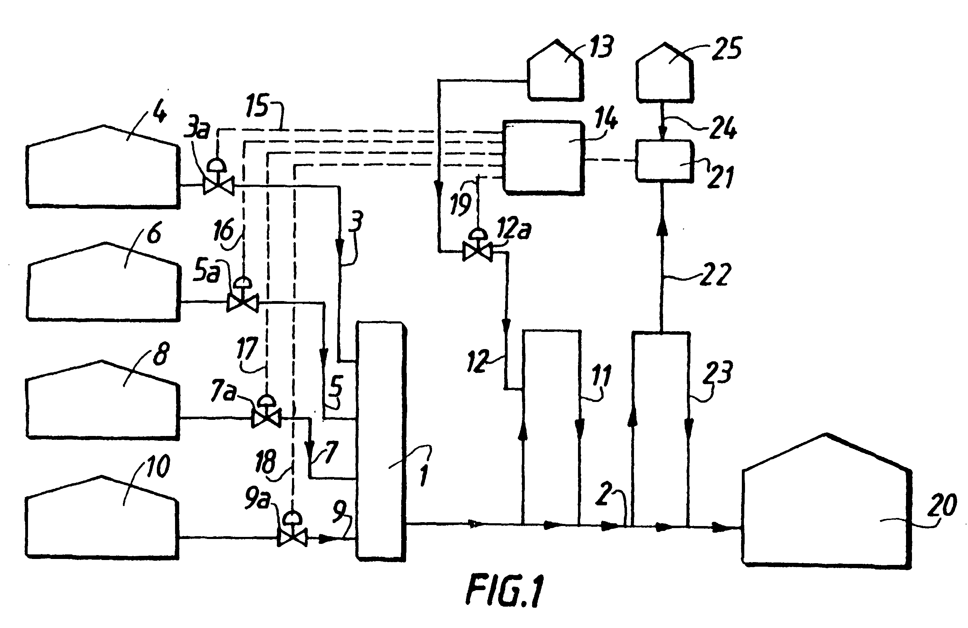

Le dispositif représenté sur la figure 1 est destiné à la réalisation d'un gazole présentant des propriétés prédéterminées, et notamment un nombre de cétane préfixé, par mélange en ligne de divers constituants dans une mélangeuse 1 et, éventuellement, dans la ligne 2 d'évacuation de ce mélange vers un réservoir 20. Dans le cas présent, la mélangeuse 1 est alimentée par quatre lignes (3,5,7,9) équipées chacune d'une vanne automatique de réglage de débit (3a, 5a, 7a 9a), en divers constituants du mélange, issus de réservoirs de stockage (4, 6, 8, 10). Ces divers constituants, dont l'un doit avoir nécessairement un nombre de cétane inférieur à la valeur de consigne, peuvent être, par exemple et sans caractère limitatif, une essence lourde issue d'un craqueur catalytique, une fraction légère issue d'une tour de distillation de pétrole brut, un gazole à haute teneur en soufre, ou un gazole à basse teneur en soufre issu d'une unité de désulfuration des gazoles.

Dans un circuit en boucle 11, monté en dérivation sur la ligne d'évacuation 2, peut être injecté un additif dit procétane, comme, par exemple, le 2-éthyl-hexyl-nitrate, par l'intermédiaire d'une ligne 12, équipée d'une vanne automatique de réglage de débit 12a, à partir d'un réservoir 13.

Ainsi qu'il a été exposé ci-dessus, divers analyseurs en ligne (non représentés) sont connectés de façon connue à la ligne 2 en aval de l'injection de procétane, pour mesurer à intervalles réguliers les propriétés caractéristiques du mélange et pour comparer les valeurs mesurées aux valeurs de consigne de ces propriétés. Ces analyseurs sont reliés à un ordinateur 14, connecté par des lignes 15, 16, 17, 18 et 19, aux vannes: 3a, 5a, 7a, 9a et 12a, et programmé de manière à régler les débits commandés par ces vannes de manière à réduire l'écart entre les valeurs mesurées et les valeurs de consigne.

Conformément à l'invention, le nombre de cétane du mélange évacué par la ligne 2 est également mesuré en ligne et les débits d'alimentation de la mélangeuse 1 et d'injection du procétane dans la ligne 2 sont ajustés de manière à rapprocher le nombre de cétane mesuré de la valeur de consigne désirée pour ce dit nombre.

A cet effet, un dispositif 21 de mesure du délai d'inflammation du mélange en cours de fabrication, et du délai d'inflammation d'un produit présentant un nombre de cétane connu et proche de la valeur de consigne, est alimenté:

- d'une part, en mélange à tester, par une première ligne 22, connectée à un circuit en boucle 23 de prélèvement d'un échantillon, monté en dérivation sur la ligne 2, en aval de l'injection du procétane ;
- d'autre part, par une seconde ligne 24, à partir d'un réservoir 25, en un produit ayant un nombre de cétane connu et proche de la valeur de consigne de la fabrication désirée.

Le dispositif 21 est lui-même connecté à l'ordinateur 14 et lui transmet un signal reflétant la différence entre le nombre de cétane connu mesuré et le nombre de cétane de l'échantillon du mélange, de manière que l'ordinateur puisse régler en conséquence les débits des différents constituants.

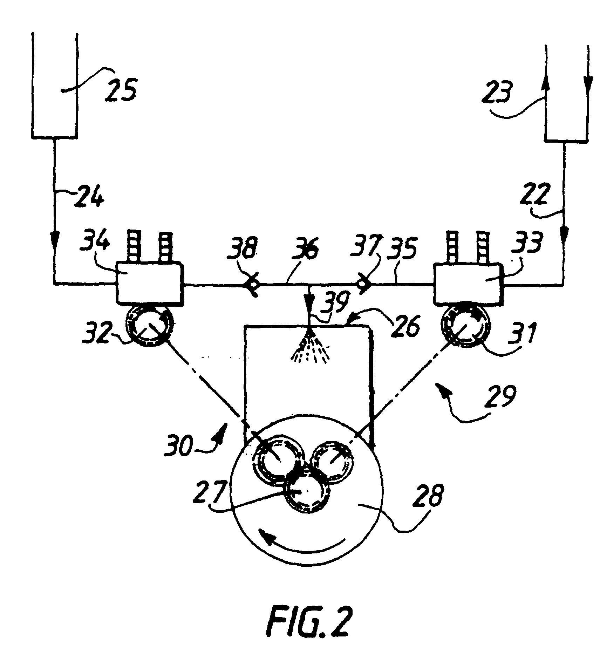

Le dispositif 21 est représenté en détail sur la figure 2. Il comprend un moteur 26 de type C.F.R. fonctionnant à taux de compression constant et avec une avance fixe, par exemple de 13° par rapport au point mort haut (avance fixée par la norme ASTM D 613). Le moteur 26 est alimenté de façon commandée par les lignes 22 et 24 et il entraîne en rotation un arbre 27, muni d'un volant 28. L'arbre 27 est lui-même couplé, par des systèmes de transmission 29, 30, par exemple à pignons, aux deux pompes à haute pression 33, 34, disposées respectivement sur la ligne 22 d'alimentation en mélange à mesurer et sur la ligne 24 d'alimentation en produit à nombre de cétane connu. Ces pompes sont connectées par des lignes, respectivement 35, 36, munies d'un clapet anti-retour, respectivement 37, 38, à une ligne d'injection 39, connectée à l'injecteur du moteur.

Comme expliqué ci-dessus, le moteur 26 est équipé d'un système de commande automatique, de manière à alimenter à intervalles réguliers, successivement, le moteur avec l'échantillon de mélange à mesurer, puis avec le produit de référence de nombre de cétane connu.

La différence de réponse du moteur pour ces deux produits est convertie par un moyen de calcul en écart du nombre de cétane, puis transmise sous forme d'un signal électrique à l'ordinateur 14. Celui-ci calcule le nombre de cétane de l'échantillon du mélange, en appliquant les règles de la norme ASTM D 2885, compare la valeur obtenue à la valeur de consigne fixée pour le mélange et ajuste en conséquence les débits d'alimentation des différents constituants du mélange ou l'injection de l'additif de procétane.

Seule la ligne 39, de faible longueur, est commune aux deux circuits d'alimentation du moteur 26, de sorte qu'après une mesure, il n'est pas nécessaire de purger le circuit et la pompe venant d'être utilisés, avant d'effectuer la mesure suivante par le second circuit et la seconde pompe. Ceci constitue un avantage considérable par rapport à la méthode ASTM D 613, qui utilise une unique pompe et deux produits de référence ayant des nombres de cétane connus.

L'invention apporte donc, pour la première fois, un système de mesure du nombre de cétane utilisable en ligne dans un ensemble de préparation d'un carburant, notamment pour moteur diesel, par mélange en ligne des constituants de ce carburant.

**Revendications**

1. Procédé de préparation d'un carburant, notamment pour moteur diesel, ou d'un combustible pour installation de chauffage, par mélange en ligne de ses constituants, avec éventuellement incorporation d'additifs, ce carburant devant posséder en vue de sa commercialisation un nombre de cétane spécifique, procédé dans lequel on alimente une mélangeuse (1) en continu avec les différents constituants (3-9) à des débits commandés (3a-9a), on mesure, à la sortie de la mélangeuse (1) et à différents intervalles de temps, le nombre de cétane du carburant en cours de préparation, on calcule l'écart de ce nombre de cétane par rapport à au moins une valeur de consigne, puis on ajuste les débits respectifs des différents constituants (3a-9a) ou d'additifs (12a) de manière à annuler l'écart entre les valeurs mesurées et les valeurs de consigne, ce procédé étant **caractérisé en ce que** l'on alimente un moteur de type " C.F.R. " (26), fonctionnant avec un taux de compression constant, alternativement avec un carburant en cours de fabrication (22) prélevé sur la ligne d'évacuation (2) de la mélangeuse (1), puis, par une ligne distincte (24), avec un produit de référence (25) dont le nombre de cétane est connu, les deux circuits d'alimentation (22,24) étant distincts et possédant chacun une pompe d'injection haute pression (33,34) distincte, permettant l'auto-inflammation du carburant.

2. Procédé selon la revendication 1, **caractérisé en ce que** la durée des mesures sur le carburant en cours de fabrication puis sur le produit de référence est inférieure à une heure, et de préférence inférieure à dix minutes.

3. Procédé selon l'une des revendications 1 et 2, **caractérisé en ce que** l'on mesure séquentiellement, dans les mêmes conditions et à l'aide d'un même moteur C.F.R. (26), le délai d'inflammation d'un échantillon du mélange en cours de fabrication, puis le délai d'inflammation d'un produit de référence ayant un nombre de cétane connu.

4. Procédé selon l'une des revendications 1 à 3, **caractérisé en ce que** le produit de référence utilisé aura un nombre de cétane proche de celui recherché pour le mélange en cours de fabrication, et dont l'écart avec la valeur de consigne est inférieur à 5 points de cétane et de préférence inférieur à 2.

5. Procédé selon l'une des revendications 1 à 4, **caractérisé en ce que** l'échantillon du mélange et le produit de référence qui alimentent le moteur C.F. R. sont introduits par l'intermédiaire de circuits distincts ou n'ayant qu'une faible partie commune dans la même zone d'injection du dit moteur.

6. Procédé selon l'une des revendications 1 à 5, **caractérisé en ce qu'**il comporte une phase d'addition en ligne, en quantités réglables, d'un additif de procétane, dans la mélangeuse, ou en amont de celle-ci, ou encore en aval de celle-ci.

7. Dispositif pour la préparation d'un carburant notamment pour moteur diesel, ou un combustible pour installation de chauffage, par mélange en ligne de ses constituants avec éventuellement incorporation d'additifs, ce carburant devant posséder en vue de sa commercialisation un nombre de cétane spécifique, ce dispositif comprenant au moins deux bacs (4-10) pour le stockage d'au moins deux constituants du mélange, une mélangeuse (1) alimentée par des lignes distinctes (3-9) à débits réglables en les divers constituants du mélange, au moins une ligne d'évacuation (2) du mélange réalisé dans la mélangeuse (1), de préférence un bac (20) pour le stockage du produit fabriqué, éventuellement une source d'additifs de procétane (13) à débit réglable et automatisé, connectée en amont ou en aval à la mélangeuse (1) et/ou à la ligne d'évacuation (2), un moyen de mesure du nombre de cétane (21), au moins un moyen (14) de commande du débit des diverses lignes (3-9) d'alimentation de la mélangeuse (1) et de la ligne (12) d'alimentation en procétane, ce moyen de commande (14) étant d'une part, asservi au moyen de comparaison et d'autre part, programmé de manière à ajuster les différents débits pour réduire l'écart entre les valeurs mesurées et les valeurs du nombre de cétane de consigne, ce dispositif étant **caractérisé en ce que** :

- le moyen de mesure (21) destiné à mesurer le nombre de cétane du mélange comprend un moteur (26) du type C.F.R. monté de préférence en dérivation sur la ligne d'évacuation (2) du mélange,
- le moteur (26) C.F.R. est connecté respectivement par des lignes distinctes (22,35; 24,36), équipées d'un moyen d'obturation commandé, à ladite ligne (2) d'évacuation et à une source (25) d'un produit de référence ayant un nombre de cétane connu,
- le moteur (26) C.F.R. est pré-réglé pour fonctionner à taux de compression constant et avec une avance à l'injection réglée sur une valeur prédéterminée,
- le moteur (26) C.F.R. est apte à fournir au moyen (14) de commande des débits des différentes lignes d'alimentation en constituants du mélange, ou éventuellement en additif, un signal représentatif de la différence entre deux mesures du nombre de cétane, les dites mesures étant effectuées successivement sur le mélange et sur le produit de référence ou inversement.

8. Dispositif selon la revendication 7, **caractérisé en ce que** les lignes (22, 35; 24, 36) d'alimentation du moteur (26) C.F.R. en échantillon du mélange à tester et en produit à- nombre de cétane connu sont distinctes ou ne comprennent qu'une faible partie commune.

9. Dispositif selon l'une des revendications 7 et 8, **caractérisé en ce que** les lignes (22, 35; 24, 36) d'alimentation du moteur (26) C.F.R. en échantillon du mélange à tester et en produit à nombre de cétane connu comprennent chacune une pompe à injection haute pression.

10. Dispositif selon l'une des revendications 7 à 9, **caractérisé en ce que** les lignes (22, 35; 24, 36) d'alimentation du moteur (26) C.F.R. en échantillon du mélange à tester et en produit à nombre de cétane connu comprennent un clapet anti-retour (37, 38).

11. Dispositif selon l'une des revendications 7 à 10, **caractérisé en ce qu'**il comprend un moyen de commande à intervalles réguliers des pompes d'alimentation (33, 34).

12. Dispositif selon l'une des revendications 7 à 11, **caractérisé en ce que** le moteur C.F.R. a été étalonné à l'aide d'au moins deux étalons ayant des nombres de cétane encadrant le nombre de cétane du produit de référence.

**Patentansprüche**

1. Verfahren zum Bereiten eines Kraftstoffs, insbesondere für einen Dieselmotor, oder eines Brennstoff für eine Heizungsanlage durch In-Line-Vermischung seiner Bestandteile, eventuell mit Einbringen von Zusätzen, wobei der Kraftstoff im Hinblick auf seine Vermarktung eine bestimmte Cetanzahl aufweisen soll,

   wobei in dem Verfahren:

   einem Mischer (1) fortlaufend verschiedene Bestandteile (3-9) mit gesteuerten Durchflüssen (3a-9a) zugeführt werden,
   am Ausgang des Mischers (1) in verschiedenen Zeitintervallen die Cetanzahl des Kraftstoffs im Verlauf der Bereitung gemessen wird,
   die Abweichung dieser Cetanzahl gegenüber zumindest einem Sollwert berechnet wird,
   daraufhin die jeweiligen Durchflüsse der verschiedenen Bestandteile (3a-9a) oder Zusätze (12a) so eingestellt werden, dass die Abweichung zwischen den Messwerten und den Sollwerten beseitigt wird,

   wobei das Verfahren **dadurch gekennzeichnet ist, dass**
   einem Motor vom CFR-Typ (26), der mit einem konstanten Kompressionsverhältnis arbeitet, abwechselnd ein in der Herstellung befindlicher Kraftstoff (22), der der Ablassleitung (2) des Mischers (1) entnommen ist, und dann über eine andere Leitung (24) ein Referenzprodukt (25), dessen Cetanzahl bekannt ist, zugeführt werden,
   wobei die zwei Zuführkreise (22, 24) verschieden sind und jeweils eine verschiedene Hochdruckeinspritzpumpe besitzen, die die Selbstentflammung des Kraftstoffs ermöglichen.

2. Verfahren nach Anspruch 1, **dadurch gekennzeichnet, dass** die Dauer der Messungen an dem in der Herstellung befindlichen Kraftstoff und dann an dem Referenzprodukt kleiner als eine Stunde und vorzugsweise kleiner als zehn Minuten ist.

3. Verfahren nach Anspruch 1 oder 2, **dadurch gekennzeichnet, dass** nacheinander unter denselben Bedingungen und mit Hilfe desselben CFR-Motors (26) die Entflammungsverzögerung einer Probe der in der Herstellung befindlichen Mischung und dann die Entflammungsverzögerung eines Referenzprodukts mit bekannter Cetanzahl gemessen werden.

4. Verfahren nach einem der Ansprüche 1 bis 3, **dadurch gekennzeichnet, dass** das verwendete Referenzprodukt eine Cetanzahl aufweist, die nahe bei der für die in der Herstellung befindliche Mischung gesuchten liegt und deren Abweichung von dem Sollwert kleiner als 5 Cetanpunkte und vorzugsweise kleiner als 2 Cetanpunkte ist.

5. Verfahren nach einem der Ansprüche 1 bis 4, **dadurch gekennzeichnet, dass** die Probe der Mischung und das Referenzprodukt, die den CFR-Motor speisen, über verschiedene Kreise oder über Kreise eingeführt werden, die nur einen geringen Abschnitt in derselben Einspritzzone des Motors gemeinsam haben.

6. Verfahren nach einem der Ansprüche 1 bis 5, **dadurch gekennzeichnet, dass** es eine Phase der In-Line-Hinzufügung eines Zusatzes von Procetan in einstellbaren Mengen in dem Mischer oder vor oder hinter diesem enthält.

7. Vorrichtung zum Bereiten eines Kraftstoffs, insbesondere für einen Dieselmotor, oder eines Brennstoff für eine Heizungsanlage durch In-Line-Vermischung seiner Bestandteile, eventuell mit Einbringen von Zusätzen, wobei der Kraftstoff im Hinblick auf seine Vermarktung eine bestimmte Cetanzahl aufweisen soll,

   wobei die Vorrichtung enthält:

   zumindest zwei Behälter (4-10) zur Speicherung von zumindest zwei Mischungsbestandteilen,
   einen Mischer (1), der von verschiedenen Leitungen (3-9) mit einstellbaren Durchflüssen für verschiedene Mischungsbestandteile gespeist wird,
   zumindest eine Ablassleitung (2) für die in dem Mischer (1) erstellte Mischung
   vorzugsweise einen Behälter (20) zur Speicherung des hergestellten Produkts,
   eventuell eine Quelle für Procetanzusätze (13) mit einstellbarem und automatisiertem Durchfluss, die vor oder hinter dem Mischer (1) oder an die Ablassleitung (2) angeschlossen ist,
   ein Messmittel für die Cetanzahl (21),
   zumindest ein Steuermittel (14) für den Durchfluss der verschiedenen Zuführleitungen (3-9) des Mischers (1) und die Zuführleitung (12) für das Procetan, wobei das Steuermittel einerseits von dem Vergleichsmittel geregelt wird und andererseits so programmiert ist, dass die unterschiedlichen Durchflüsse eingestellt werden, um die Abweichung zwischen den Messwerten und den Sollwerten für die Cetanzahl zu verringern,

   wobei die Vorrichtung **dadurch gekennzeichnet ist, dass**
   das Messmittel (21), das zum Messen der Cetanzahl der Mischung bestimmt ist, einen Motor vom CFR-Typ (26) enthält, der vorzugsweise als Abzweigung an der Ablassleitung (2) der Mischung angebracht ist,

der CFR-Motor (26) jeweils über unterschiedliche Leitungen (22,35; 24,36), die mit einem gesteuerten Verschlussmittel versehen sind, mit der Ablassleitung (2) und mit einer Quelle (25) für ein Referenzprodukt verbunden ist, das eine bekannte Cetanzahl aufweist,

der CFR-Motor (26) so voreingestellt ist, dass er mit einem konstanten Kompressionsverhältnis und mit einer auf einen vorbestimmten Wert eingestellten Einspritzzuführung arbeitet,

der CFR-Motor (26) daran angepasst ist, dem Steuermittel (14) des Durchflusses der verschiedenen Zuführleitungen für die Mischungsbestandteile oder eventuell für den Zusatz ein Signal zuzuführen, das den Unterschied zwischen zwei Messungen der Cetanzahl wiedergibt, wobei die Messungen nacheinander an der Mischung und an dem Referenzprodukt oder umgekehrt durchgeführt werden.

**8.** Vorrichtung nach Anspruch 7, **dadurch gekennzeichnet, dass** die Zuführleitungen (22,35; 24,36) des CFR-Motors (26) für die Probe der zu testenden Mischung und das Produkt mit bekannter Cetanzahl voneinander verschieden sind oder nur einen geringen Abschnitt gemeinsam haben.

**9.** Vorrichtung nach Anspruch 7 oder 8, **dadurch gekennzeichnet, dass** die Zuführleitungen (22,35; 24,36) des CFR-Motors (26) für die Probe der zu testenden Mischung und das Produkt mit bekannter Cetanzahl jeweils eine Hochdruckeinspritzpumpe enthalten.

**10.** Vorrichtung nach einem der Ansprüche 7 bis 9, **dadurch gekennzeichnet, dass** die Zuführleitungen (22,35; 24,36) des CFR-Motors (26) für die Probe der zu testenden Mischung und das Produkt mit bekannter Cetanzahl ein Rückschlagventil (37, 38) enthalten.

**11.** Vorrichtung nach einem der Ansprüche 7 bis 10, **dadurch gekennzeichnet, dass** es ein Mittel zum Steuern der Zuführpumpen (33, 34) in regelmäßigen Intervallen enthält.

**12.** Vorrichtung nach einem der Ansprüche 7 bis 11, **dadurch gekennzeichnet, dass** der CFR-Motor kalibriert wurde mit Hilfe von zumindest zwei Standards, die Cetanzahlen aufweisen, die die Cetanzahl des Referenzprodukts einrahmen.

**Claims**

**1.** A process for preparing a motor fuel, in particular for a diesel engine, or a heating system fuel, by on-line mixing of the constituents thereof, with optional incorporation of additives, said motor fuel necessarily exhibiting a specific cetane number for commercial purposes, in which process a mixer (1) is continuously supplied with the various constituents (3-9) at controlled flow rates (3a-9a), the cetane number of the in-preparation fuel is measured at the outlet of the mixer (1) and at different time intervals, the deviation of this cetane number relative to at least one setpoint value is calculated, then the respective flow rates of the various constituents (3a-9a) or additives (12a) is adjusted in such a manner as to eliminate the deviation between the measured values and the setpoint values, this process being **characterised in that** a "CFR" type engine (26), operating at a constant compression ratio, is alternately supplied with an in-production fuel (22) taken from the discharge line (2) of the mixer (1), then, via a separate line (24), with a reference product (25) having a known cetane number, the two supply circuits (22, 24) being separate and each having a high pressure injection pump (33, 34) permitting autoignition of the motor fuel.

**2.** A process according to claim 1, **characterised in that** the duration of the measurements on the in-production fuel, then on the reference product is less than one hour and preferably less than ten minutes.

**3.** A process according to either one of claims 1 and 2, **characterised in that** the ignition delay of a sample of the in-production mixture, then the ignition delay of a reference product having a known cetane number are measured sequentially under the same conditions using one and the same CFR engine (26).

**4.** A process according to any one of claims 1 to 3, **characterised in that** the reference product used will have a cetane number close to that desired for the in-production mixture and the deviation of which from the setpoint value is less than 5 and preferably less than 2 cetane points.

**5.** A process according to any one of claims 1 to 4, **characterised in that** the mixture sample and the reference product supplied to the CFR engine are introduced into the same injection zone of said engine via circuits which are separate or have only a small portion in common.

**6.** A process according to any one of claims 1 to 5, **characterised in that** it comprises an on-line addition phase of controllable quantities of a procetane additive into the mixer, or upstream therefrom; or alternatively downstream therefrom.

7. An apparatus for preparing a motor fuel, in particular for a diesel engine, or a heating system fuel, by on-line mixing of the constituents thereof, with additives optionally being incorporated, said motor fuel necessarily exhibiting a specific cetane number for commercial purposes, said apparatus comprising at least two tanks (4-10) for storage of at least two constituents of the mixture, a mixer (1) supplied by separate lines (3-9) at controllable flow rates with the various constituents of the mixture, at least one discharge line (2) of the mixture produced in the mixer (1), preferably a tank (20) for storing the manufactured product, optionally a source (13) of procetane additives with a controllable, automated flow rate connected upstream or downstream from the mixer (1) and/or to the discharge line (2), a means (21) for measuring the cetane number, at least one means (14) for controlling the flow rates of the various supply lines (3-9) to the mixer (1) and of the procetane supply line (12), said control means (14) being, on the one hand, under the control of the comparison means and, on the other, programmed to adjust the various flow rates to reduce the deviation between the measured values and the setpoint cetane number values,
said apparatus being **characterised in that**

- the measurement means (21) intended to measure the cetane number of the mixture comprises a CFR type engine (26) preferably installed in a bypass on the mixture discharge line (2),
- the CFR engine (26) is respectively connected via separate lines (22, 35; 24, 36), fitted with a controlled shut-off means, to said discharge line (2) and to a source (25) of a reference product having a known cetane number,
- the CFR engine (26) is preset to operate at a constant compression ratio and with an injection advance set to a predetermined value,
- the CFR engine (26) is capable of supplying to the control means (14) for the flow rates of the various supply lines for the mixture constituents or optionally for additive, a signal which is representative of the difference between the two cetane number measurements, said measurements being performed successively on the mixture and on the reference product or vice versa.

8. An apparatus according to claim 7, **characterised in that** the lines (22, 35; 24, 36) supplying the sample of mixture for testing and the product with a known cetane number to the CFR engine (26) are separate or have only a small portion in common.

9. An apparatus according to either one of claims 7 and 8, **characterised in that** the lines (22, 35; 24, 36) supplying the sample of mixture for testing and the product with a known cetane number to the CFR engine (26) each comprise a high pressure injection pump.

10. An apparatus according to any one of claims 7 to 9, **characterised in that** the lines (22, 35; 24, 36) supplying the sample of mixture for testing and the product with a known cetane number to the CFR engine (26) comprise a nonreturn valve (37, 38).

11. An apparatus according to any one of claims 7 to 10, **characterised in that** it comprises a means for actuating the supply pumps (33, 34) at regular intervals.

12. An apparatus according to any one of claims 7 to 11, **characterised in that** the CFR engine has been calibrated by means of at least two standards having cetane numbers which bracket the cetane number of the reference product.

FIG.1

EP 0 894 268 B1

FIG.2